# EUROPÄISCHE PATENTANMELDUNG

(11) **EP 1 950 196 A1**
(43) Veröffentlichungstag der Anmeldung: **30.07.2008**
(21) Anmeldenummer: 07101364.3
(22) Anmeldetag: 29.01.2007
(51) Int. Cl.: C07C 215/40, C07D 211/74, C07D 451/06, C07D 491/18

(54) **Verfahren zur Herstellung von Ammoniumhexafluorophosphaten**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die Erfindung betrifft ein Verfahren zur Herstellung von Ammoniumhexafluorophosphaten der allgemeinen Formel **1** 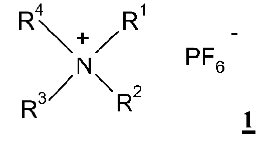 worin R¹, R², R³ und R⁴ die in den Ansprüchen und in der Beschreibung genannten Bedeutungen haben kann, neue Ammoniumhexafluorophosphate als solche sowie deren Verwendung zur Herstellung pharmazeutisch wirksamer Verbindungen.

## Beschreibung

Die Erfindung betrifft ein Verfahren zur Herstellung von Ammoniumhexafluorophosphaten der allgemeinen Formel **1** 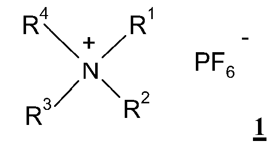 worin R¹, R², R³ und R⁴ die in den Ansprüchen und in der Beschreibung genannten Bedeutungen haben kann, neue Ammoniumhexafluorophosphate als solche sowie deren Verwendung zur Herstellung pharmazeutisch wirksamer Verbindungen.

### Beschreibung der Erfindung

Die vorliegende Erfindung betrifft ein Verfahren zur Herstellung von Ammoniumhexafluorophosphaten der Formel **1** 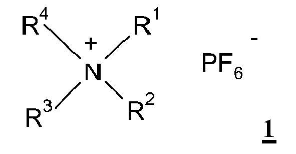 worin
- R¹ und R²: gleich oder verschieden Wasserstoff oder ein Rest ausgewählt aus der Gruppe bestehend C₁-C₁₀-Alkyl, C₂-C₁₀-Alkenyl, C₂-C₁₀-Alkinyl, C₃-C₈-Cycloalkyl, C₄-C₈-Cycloalkenyl, C₆-C₈-Cycloalkinyl, C₆-C₁₀-Aryl-C₁-C₆-alkyl, C₆-C₁₀-Aryl-C₂-C₆-alkenyl, C₆-C₁₀-Aryl-C₂-C₆-alkinyl, C₆-C₁₀-Aryl und Heterocyclyl, der gegebenenfalls substituiert sein kann;
- R³ und R⁴: gleich oder verschieden Wasserstoff oder ein Rest ausgewählt aus der Gruppe bestehend C₁-C₁₀-Alkyl, C₂-C₁₀-Alkenyl, C₂-C₁₀-Alkinyl, C₃-C₈-Cycloalkyl, C₄-C₈-Cycloalkenyl, C₆-C₈-Cycloalkinyl, C₆-C₁₀-Aryl-C₁-C₆-alkyl, C₆-C₁₀-Aryl-C₂-C₆-alkenyl, C₆-C₁₀-Aryl-C₂-C₆-alkinyl, C₆-C₁₀-Aryl und Heteroaryl, der gegebenenfalls substituiert sein kann, oder
- R³ und R⁴: gemeinsam mit dem Stickstoff ein mono-, bi- oder tricyclischer, gesättigter oder ungesättigter Carbocyclus der 4 bis 10 Kohlenstoffzentren enthalten kann, wobei gegebenenfalls ein oder zwei dieser Kohlenstoffzentren durch O oder S ersetzt sein können, und der gegebenenfalls substituiert sein kann;
dadurch gekennzeichnet, dass eine Verbindung der Formel 2 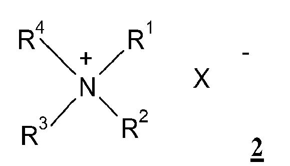 in der R¹, R², R³ und R⁴ die vorstehend für Verbindung **1** genannten Bedeutungen haben und worin
- X⁻: ein einfach negativ geladenes Anion bedeuten kann,
in einem geeigneten Lösemittel durch Umsetzung mit einem Salz Kat⁺PF⁶⁻, wobei Kat⁺ für ein Kation ausgewählt aus der Gruppe bestehend aus Li⁺, Na⁺, K⁺, Mg2+, Ca2+, steht, in die Verbindung der Formel **1** überführt wird,
mit der Maßgabe, dass die Verbindung der Formel **1** nicht die Verbindung der Formel **1'** 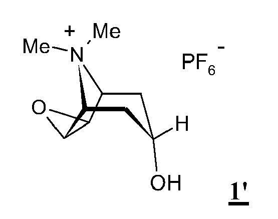 sein kann.

Ein erfindungsgemäß besonders bevorzugtes Verfahren ist dadurch gekennzeichnet, dass die Umsetzung der Verbindung der Formel **2** zur Verbindung der Formel **1** mit Hilfe eines Salzes Kat⁺PF₆⁻ erfolgt, in dem Kat⁺ ausgewählt ist aus der Gruppe bestehend aus Li⁺, Na⁺ und K⁺, besonders bevorzugt Na⁺ und K⁺. Im Rahmen der vorliegenden Erfindung werden die Salze Salzes Kat⁺PF₆⁻ gegebenenfalls auch als Salze Salzes KatPF₆ bezeichnet.

Als Lösungsmittel zur Durchführung des erfindungsgemäßen Verfahrens kommen bevorzugt polare Lösemittel in Betracht. Bevorzugte Lösemittel sind erfindungsgemäß ausgewählt aus der Gruppe bestehend aus Wasser, Methanol, Ethanol, Propanol, Isopropanol und Mischungen davon, wobei Wasser, Methanol und Mischungen davon erfindungsgemäß herausragende Bedeutung zukommt.

Pro Mol eingesetzte Verbindung der Formel **2** werden erfindungsgemäß bevorzugt 1 Mol, bevorzugt 1-1.5 Mol, gegebenenfalls auch 2-5 Mol des Salzes KatPF₆ eingesetzt. Für den Fachmann ist ersichtlich, dass der Einsatz geringerer Mengen an Salz KatPF₆ möglich ist, dass dies aber dann nur zu einer partiellen Umsetzung der Verbindung der Formel **2** führen kann.

Das erfindungsgemäße Verfahren wird bevorzugt unter milden Reaktionsbedingungen, das heißt bei Temperaturen im Bereich von 10-55°C, besonders bevorzugt 15-50°C, besonders bevorzugt 20-45°C, durchgeführt. Nach vollständiger Zugabe der Salze KatPF₆, teilweise auch schon während der Zugabe, kristallisieren die Verbindungen der Formel **1** aus der Lösung aus. Die erhaltenen Produkte können, falls erforderlich, durch Umkristallisation aus einem der vorstehend genannten Lösemittel gereinigt werden. Die erhaltenen Kristalle werden isoliert und im Vakuum getrocknet.

Erfindungsgemäß bevorzugt sind Verfahren zur Herstellung der Verbindungen der Formel **1** worin
- R¹ und R²: gleich oder verschieden Wasserstoff oder ein Rest ausgewählt aus der Gruppe bestehend C₁-C₆-Alkyl, C₂-C₆-Alkenyl, C₂-C₆-Alkinyl, C₃-C₆-Cycloalkyl, C₆-C₁₀-Aryl-C₁-C₄-alkyl, und C₆-C₁₀-Aryl, der gegebenenfalls substituiert sein kann durch einen oder mehrere Reste ausgewählt aus der Gruppe bestehend aus OH, F, Cl, Br, =O, CN, NO₂, -C₁-C₄-Alkoxy und -COOC₁-C₄-Alkyl;
- R¹ und R²: gleich oder verschieden ein Rest ausgewählt aus der Gruppe bestehend C₁-C₆-Alkyl, C₂-C₆-Alkenyl, C₂-C₆-Alkinyl, C₃-C₆-Cycloalkyl, C₆-C₁₀-Aryl-C₁-C₄-alkyl, und C₆-C₁₀-Aryl, der gegebenenfalls substituiert sein kann durch einen oder mehrere Reste ausgewählt aus der Gruppe bestehend aus OH, F, Cl, Br, =O, CN, NO₂, -C₁-C₄-Alkoxy und -COOC₁-C₄-Alkyl, oder
- R³ und R⁴: gemeinsam mit dem Stickstoff ein mono-, bi- oder tricyclischer, gesättigter oder ungesättigter Carbocyclus der 4 bis 10 Kohlenstoffzentren enthalten kann, wobei gegebenenfalls ein oder zwei dieser Kohlenstoffzentren durch O ersetzt sein können, und der gegebenenfalls substituiert sein kann durch einen oder mehrere Reste ausgewählt aus der Gruppe bestehend aus OH, F, Cl, Br, =O, CN, NO₂, -C₁-C₄-Alkoxy, C₁-C₄-Alkyl, -COOC₁-C₄-Alkyl, und -O-COR', wobei
R' ein Rest ausgewählt aus der Gruppe bestehend aus C₁-C₄-Alkyl, C₂-C₆-Alkenyl und C₁-C₄-alkylen-Phenyl, die jeweils substituiert sein können durch Hydroxy, Hydroxymethyl oder C₁-C₄-Alkoxy,
mit der Maßgabe, dass die Verbindung der Formel **1** nicht die Verbindung der Formel **1'** 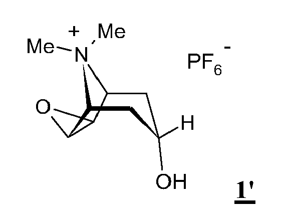 sein kann.

Erfindungsgemäß bevorzugt sind ferner Verfahren zur Herstellung der Verbindungen der Formel **1** worin
- R¹ und R²: gleich oder verschieden Wasserstoff oder ein Rest ausgewählt aus der Gruppe bestehend C₁-C₆-Alkyl, C₂-C₆-Alkenyl, Phenylethyl, Benzyl und Phenyl, der gegebenenfalls substituiert sein kann durch einen oder mehrere Reste ausgewählt aus der Gruppe bestehend aus OH, F, Cl, Br, =O, CN, NO₂, Methoxy, Ethoxy und -COOMe;
- R³ und R⁴: gleich oder verschieden Wasserstoff oder ein Rest ausgewählt aus der Gruppe bestehend C₁-C₆-Alkyl, C₂-C₆-Alkenyl, Phenylethyl, Benzyl und Phenyl, der gegebenenfalls substituiert sein kann durch einen oder mehrere Reste ausgewählt aus der Gruppe bestehend aus OH, F, Cl, Br, =O, CN, NO₂, Methoxy, Ethoxy und -COOMe,
oder
- R³ und R⁴: gemeinsam mit dem Stickstoff eine Gruppe ausgewählt aus Pyrrol, Pyrrolin, Pyrrolidin, Pyridin, Piperidin, Morpholin, 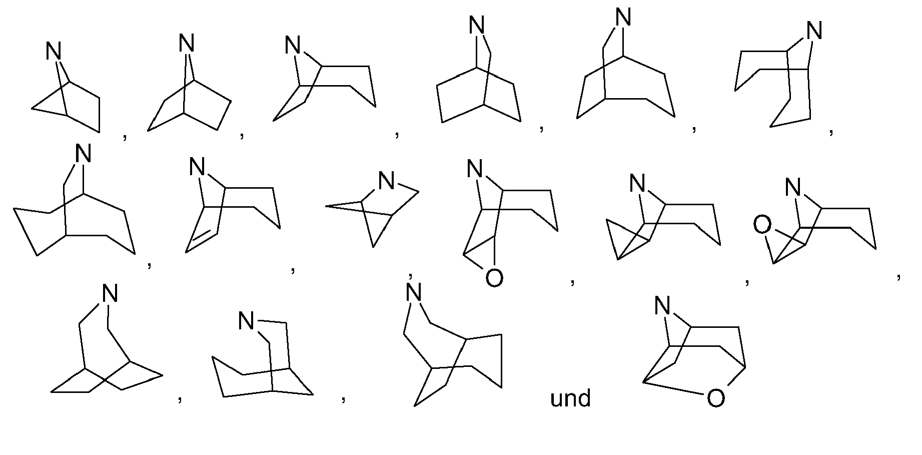 bilden, die gegebenenfalls substituiert sein kann durch einen oder mehrere, bevorzugt einen Rest ausgewählt aus der Gruppe bestehend aus OH, F, =O, Methyl, Ethyl, Methoxy und -O-COR',
wobei
R' ein Rest ausgewählt aus der Gruppe bestehend aus C₁-C₄-Alkyl, Benzyl und Phenylethyl, der jeweils substituiert sein kann durch Hydroxy, Hydroxymethyl oder Methoxy;
mit der Maßgabe, dass die Verbindung der Formel **1** nicht die Verbindung der Formel **1'** 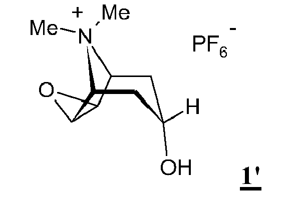 sein kann.

Erfindungsgemäß bevorzugt sind ferner Verfahren zur Herstellung der Verbindungen der Formel **1** worin
- R¹ und R²: gleich oder verschieden Wasserstoff oder ein Rest ausgewählt aus der Gruppe bestehend C₁-C₄-Alkyl, C₂-C₄-Alkenyl, Phenylethyl, Benzyl und Phenyl, der gegebenenfalls substituiert sein kann durch einen oder mehrere Reste ausgewählt aus der Gruppe bestehend aus OH, F, Cl, Br, =O, CN, NO₂, Methoxy, Ethoxy und -COOMe;
bedeuten und die Reste R³ und R⁴ die vor- oder nachstehend genannten Bedeutungen haben können.

Erfindungsgemäß bevorzugt sind ferner Verfahren zur Herstellung der Verbindungen der Formel **1** worin
- R¹ und R²: gleich oder verschieden Wasserstoff oder ein Rest ausgewählt aus der Gruppe bestehend Methyl, Ethyl, Propyl, Butyl, Benzyl und Phenyl, der gegebenenfalls substituiert sein kann durch einen oder mehrere Reste ausgewählt aus der Gruppe bestehend aus OH, F und =O;
bedeuten und die Reste R³ und R⁴ die vor- oder nachstehend genannten Bedeutungen haben können.

Erfindungsgemäß bevorzugt sind ferner Verfahren zur Herstellung der Verbindungen der Formel **1** worin R¹ Methyl bedeutet und R² und die Reste R³ und R⁴ die vor- oder nachstehend genannten Bedeutungen haben können. Erfindungsgemäß besonders bevorzugt sind ferner Verfahren zur Herstellung der Verbindungen der Formel **1** worin R¹ und R² Methyl bedeuten und die Reste R³ und R⁴ die vor- oder nachstehend genannten Bedeutungen haben können.

Erfindungsgemäß bevorzugt sind ferner Verfahren zur Herstellung der Verbindungen der Formel **1** worin
- R³ und R⁴: gleich oder verschieden Wasserstoff oder ein Rest ausgewählt aus der Gruppe bestehend C₁-C₄-Alkyl, C₂-C₄-Alkenyl, Phenylethyl, Benzyl und Phenyl, der gegebenenfalls substituiert sein kann durch einen oder mehrere Reste ausgewählt aus der Gruppe bestehend aus OH, F, Cl, Br, =O, CN, NO₂, Methoxy, Ethoxy und -COOMe;
bedeuten und die Reste R¹ und R² die vor- oder nachstehend genannten Bedeutungen haben können.

Erfindungsgemäß bevorzugt sind ferner Verfahren zur Herstellung der Verbindungen der Formel **1** worin
- R³ und R⁴: gleich oder verschieden Wasserstoff oder ein Rest ausgewählt aus der Gruppe bestehend Methyl, Ethyl, Propyl, Butyl, Benzyl und Phenyl, der gegebenenfalls substituiert sein kann durch einen oder mehrere Reste ausgewählt aus der Gruppe bestehend aus OH, F und =O;
bedeuten und die Reste R¹ und R² die vor- oder nachstehend genannten Bedeutungen haben können.

Erfindungsgemäß bevorzugt sind ferner Verfahren zur Herstellung der Verbindungen der Formel **1** worin R³ Methyl bedeutet und R⁴ und die Reste R¹ und R² die vor- oder nachstehend genannten Bedeutungen haben können.

Erfindungsgemäß bevorzugt sind ferner Verfahren zur Herstellung der Verbindungen der Formel **1** worin
- R³ und R⁴: gemeinsam mit dem Stickstoff eine Gruppe ausgewählt aus Pyrrol, Pyrrolin, Pyrrolidin, Pyridin, Piperidin, Morpholin, 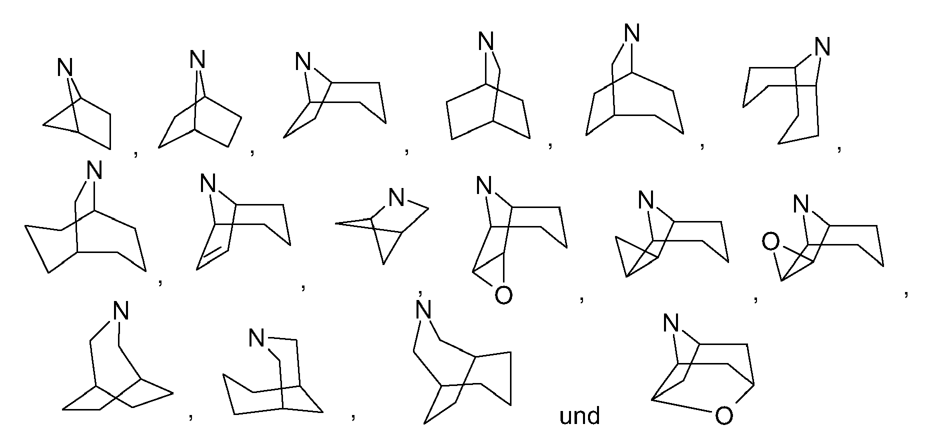 bilden, die gegebenenfalls substituiert sein kann durch einen oder mehrere, bevorzugt einen Rest ausgewählt aus der Gruppe bestehend aus OH, F, =O, Methyl, Ethyl, Methoxy und -O-COR',
wobei
R' ein Rest ausgewählt aus der Gruppe bestehend aus C₁-C₄-Alkyl, Benzyl und Phenylethyl, der jeweils substituiert sein kann durch Hydroxy, Hydroxymethyl oder Methoxy;
bedeutet und die Reste R¹ und R² die vor- oder nachstehend genannten Bedeutungen haben können.

Erfindungsgemäß bevorzugt sind ferner Verfahren zur Herstellung der Verbindungen der Formel **1** worin
- R³ und R⁴: gemeinsam mit dem Stickstoff eine Gruppe ausgewählt aus Pyrrolin, Pyrrolidin, Piperidin, 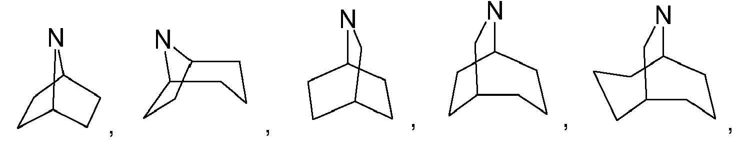 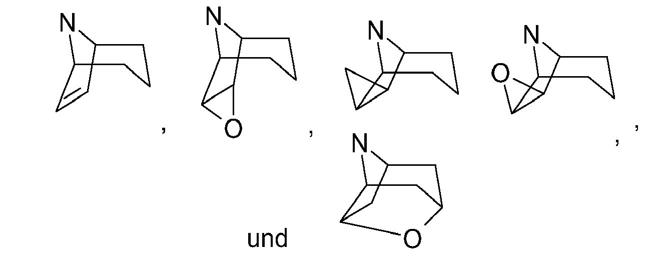 bilden, die gegebenenfalls substituiert sein kann durch einen oder mehrere, bevorzugt einen Rest ausgewählt aus der Gruppe bestehend aus OH, F, =O, Methyl, Ethyl, Methoxy und -O-COR',
wobei
R ein Rest ausgewählt aus -CH₃, -CH₂-CH₃, -CH₂-CH₂-OH, -CH(OH)-CH₃, -CH₂-Phenyl, -CH(OH)-Phenyl und -CH(CH₂OH)-Phenyl, bevorzugt -CH₃, -CH₂-CH₃, -CH₂-Phenyl, und -CH(CH₂OH)-Phenyl, besonders bevorzugt -CH(CH₂OH)-Phenyl,
bedeutet und die Reste R¹ und R² die vor- oder nachstehend genannten Bedeutungen haben können.

Erfindungsgemäß bevorzugt sind ferner Verfahren zur Herstellung der Verbindungen der Formel **1** worin
- R³ und R⁴: gemeinsam mit dem Stickstoff eine Gruppe ausgewählt aus Pyrrolidin, Piperidin, 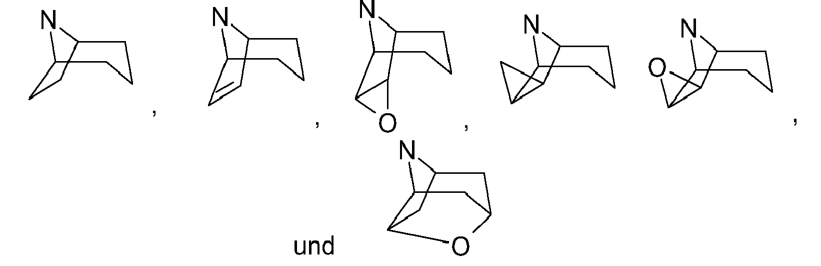 bilden, die gegebenenfalls substituiert sein kann durch einen oder mehrere, bevorzugt einen Rest ausgewählt aus der Gruppe bestehend aus OH, F, =O, Methyl, Ethyl, Methoxy und -O-COR',
wobei
R ein Rest ausgewählt aus -CH₃, -CH₂-CH₃, -CH₂-CH₂-OH, -CH(OH)-CH₃, -CH₂-Phenyl, -CH(OH)-Phenyl und -CH(CH₂OH)-Phenyl, bevorzugt -CH₃, -CH₂-CH₃, -CH₂-Phenyl, und -CH(CH₂OH)-Phenyl, besonders bevorzugt -CH(CH₂OH)-Phenyl,
bedeutet und die Reste R¹ und R² die vor- oder nachstehend genannten Bedeutungen haben können.

Als Alkylgruppen sowie Alkylgruppen, welche Bestandteil anderer Reste sind, werden verzweigte und unverzweigte Alkylgruppen mit 1 bis 10 Kohlenstoffatomen bezeichnet. Beispielsweise werden genannt: Methyl, Ethyl, Propyl, Butyl. Sofern nicht anders genannt, sind von den vorstehend genannten Bezeichnungen Propyl, Butyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl.

Als Alkoxy- oder Alkyloxygruppen, werden verzweigte und unverzweigte Alkylgruppen mit 1 bis 10 Kohlenstoffatomen bezeichnet, die über ein Sauerstoffatom verknüpft sind. Beispielsweise werden genannt: Methoxy, Ethoxy, Propoxy, Butoxy. Sofern nicht anders genannt, sind von den vorstehend genannten Bezeichnungen sämtliche der möglichen isomeren Formen umfaßt.

Als Alkenylgruppen sowie Alkenylgruppen, welche Bestandteil anderer Reste sind, werden verzweigte und unverzweigte Alkylgruppen mit 1 bis 10 Kohlenstoffatomen bezeichnet, soweit sie wenigstens eine Doppelbindung enthalten.

Als Alkinylgruppen sowie Alkinylgruppen, welche Bestandteil anderer Reste sind, werden verzweigte und unverzweigte Alkylgruppen mit 1 bis 10 Kohlenstoffatomen bezeichnet, soweit sie wenigstens eine Dreifachbinsung enthalten.

Als Cycloalkylreste mit 3 - 8 Kohlenstoffatomen werden cyclische Alkylgruppen wie beispielsweise Cyclopropyl, Cyclobutyl, Cyclopentyl oder Cyclohexyl bezeichnet.

Als Cycloalkenylreste mit 4 - 8 Kohlenstoffatomen werden Cycloalkylgruppen verstanden, soweit sie wenigstens eine Doppelbindung aufweisen.

Als Cycloalkenylreste mit 6 - 8 Kohlenstoffatomen werden Cycloalkylgruppen verstanden, soweit sie wenigstens eine Dreifachbindung aufweisen.

Als Arylgruppen werden aromatische Ringsysteme mit 6 bis 10 Kohelnstoffatomen verstanden. Bevorzugte Arylgruppen sind Phenyl und Naphthyl wobeio Phenyl besondere Bedeutung zukommt.

Aryl-alkyl-gruppen sind Arylgruppen, die über Alkylgruppen verknüpft sind. Bevorzugte Arylalkylgruppen sind Phenylethyl und Benzyl.

Aryl-alkenyl-gruppen sind Arylgruppen, die über Alkenylgruppen verknüpft sind.

Aryl-alkinyl-gruppen sind Arylgruppen, die über Alkinylgruppen verknüpft sind.

Heterocyclyl-Gruppen sind 5- ,6- oder 7-gliedrige, gesättigte oder ungesättigte Heterocyclen, die als Heteroatome Stickstoff, Sauerstoff oder Schwefel enthalten können. Beispieslweise werden genannt Furan, Tetrahydrofuran, Tetrahydrofuranon, γ-Butylrolacton, α-Pyran, γ-Pyran, Dioxolan, Tetrahydropyran, Dioxan, Thiophen, Dihydrothiophen, Thiolan, Dithiolan, Pyrrol, Pyrrolin, Pyrrolidin, Pyrazol, Pyrazolin, Pyrazolidin, Imidazol, Imidazolin, Imidazolidin, Triazol,Tetrazol, Pyridin, Piperidin, Pyridazin, Pyrimidin, Pyrazin, Piperazin, Triazin, Tetrazin, Morpholin, Thiomorpholin, Diazepan, Oxazol, Isoxazol, Oxazin, Thiazol, Isothiazol, Thiadiazol, Oxadiazol, Pyrazo lidin.

Die Gruppe =O steht für eine Carbonylgruppe. Die Gruppe -O-CO-R' steht für eine Esterfunktion.

Die Salze quaternärer Ammoniumverbindungen , wie beispielsweise diejenigen der Formel **2** sind im allgemeinen sehr gut wasser- und alkohollöslich. Sie sind jedoch ausgesprochen schwer löslich in weniger polaren organischen Solventien wie beispielsweise Aceton, Acetonitril, Kohlenwasserstoffen, Halogenkohlenwasserstoffen oder Ethern. Chemische Umsetzungen mit quaternären Ammoniumverbindungen sind deshalb im Grundsatz auf Umsetzungen in Wasser, Alkohol oder stark polaren aprotischen Lösemitteln wie DMF (Dimethylformamid) oder NMP (N-Methylpyrrolidin) beschränkt. Daraus ergeben sich starke Einschränkungen bezüglich der Auswahl von Reaktionspartnern oder deren Abtrennung vom Zielprodukt.

Viele Synthesestrategien scheitern an der Unmöglichkeit oder Schwierigkeit, quaternäre Ammoniumverbindungen aus wässriger oder alkoholischer Lösung von anderen Reaktionskomponenten zu trennen. Mit Hilfe der Ammoniumionen der Formel **1** kann dieses Problem gelöst werden. Es gelingt die selektive Fällung oder Kristallisation der quaternären Ammoniumverbindungen der Formel **1** aus Alkoholen oder Wasser durch Umsetzung der Verbindungen **2** mit den entsprechenden Salzen KatPF₆ und damit die Isolierung und Reinigung mit regelmäßig guter Ausbeute.

Die Verbindungen **1** ermöglichen aufgrund ihrer sehr guten Löslichkeit und der ausgesprochen hohen Stabilität des Anions eine Vielzahl von Reaktionen in weniger polaren aprotischen Lösungsmitteln und können dort angewendet werden, wo Wasser oder Alkohol stört. Aufgrund dieser Eigenschaften stellen die Verbindungen der Formel **1** wertvolle Ausgangsmaterialen bei der Synthese modifizierter, quartärer Ammoniumsalze in organischen Lösemitteln dar. Nach erfolgter Umsetzung der Hexafluorophosphate zu den gewünschten modifizierten Ammoniumhexafluorophosphaten, lässt sich unter Verwendung von beispielsweise Lithiumsalzen (wie z.B. LiBr) das Hexafluorophosphat durch andere Anionen wieder ersetzen. Als Beispiel dazu kann die im experimentellen Teil der vorliegenden Erfindung beschriebene Synthese von Tiotropiumsalzen dienen.

Vor diesem Hintergrund betrifft die vorliegende Erfindung ferner Ammonium-hexafluorophosphate der allgemeinen Formel **1** als solche, 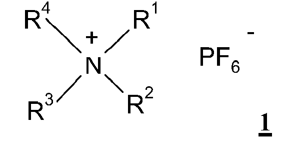 worin R', R², R³ und R⁴ die vorstehend genannten Bedeutungen haben können, mit der Maßgabe, dass es sich bei der Verbindung der Formel **1** nicht um Tiotropiumhexafluorophosphat oder um die Verbindung der Formel **1'** 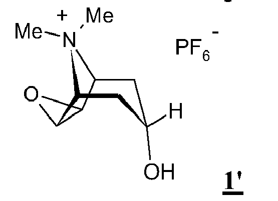 handeln kann.

Ein weiterer Aspekt der vorliegenden Erfindung betrifft die Verwendung der vorstehend genannten Hexafluorophosphate der Formel **1** als Ausgangsverbindungen zur Herstellung von Ammoniumsalzen.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken.

### Synthesebeispiele - Allgemeine Arbeitsvorschrift:

Eine Ammoniumverbindung der Formel **2** wird in Wasser gelöst und mit einer equimolaren oder molar überschüssigen Menge eines in Wasser löslichen Hexafluorophosphates (Natrium- oder Kaliumsalz) versetzt.
Das Hexafluorophosphat der Formel **1** fällt bzw. kristallisiert als weißes, wasserunlösliches Produkt aus, wird isoliert, gegebenenfalls mit Methanol gewaschen und dann bei ca. 40°C im Trockenschrank getrocknet.

In Analogie zu vorstehender allgemeiner Arbeitsvorschrift wurden die folgenden Verbindungen erhalten.

### Beispiel 1: N-Methyltropinium hexafluorophosphat

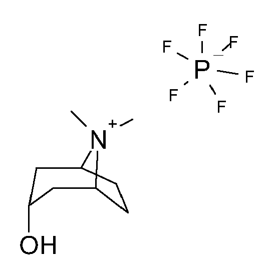

18,4g N-Methyltropinium iodid werden in 50 ml Wasser gelöst und durch Zugeben einer Lösung aus 11,4 g NaPF6 30 ml Wasser zur Kristallisation gebracht. Die Kristalle werden filtriert, mit Wasser gewaschen und getrocknet. Ausbeute: 19,6 g (74%)

### Beispiel 2: N-Methylscopolinium hexafluorophosphat

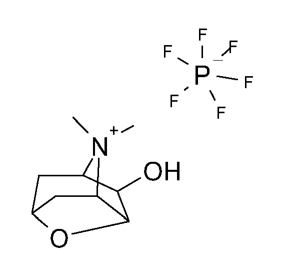

N-Methylscopinium Bromid (20 g) wird in Methanol (100 ml) gelöst unter Zusatz einer katalyt. Menge (4-14 mol% ) Natriummethylat unter Rückfluß zur Reaktion (Umlagerung) gebracht und anschließend mit einer equimolaren oder molar überschüssigen Menge einer Lösung aus Natriumhexafluorophosphat (13g) in 33 ml Methanol versetzt.

Das N-Methylscopolinium-hexafluorophosphat fällt / kristallisiert als weißes, wenig wasserlösliches Produkt aus, wird isoliert, ggf. mit Methanol gewaschen und dann bei ca. 40°C im Vakuum getrocknet. Ausbeute : 25g (72%); Fp: 292-293°C

### Beispiel 3: N-Methyl-endo-epoxy-tropenonium hexafluorophosphat

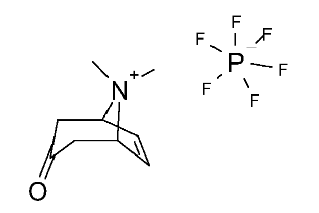

1 g N-Methyl-endo-epoxy-tropenonium Bromid werden in 25 ml Wasser gelöst und durch Zugeben einer Lösung aus 0,62 g Natriumhexafluorophosphat in 10 ml Wasser zur Kristallisation gebracht. Die Kristalle werden filtriert, mit Wasser gewaschen und getrocknet. Ausbeute: 1,3 g

### Beispiel 4: N,N-Dimethylpiperidonium hexafluorophosphat

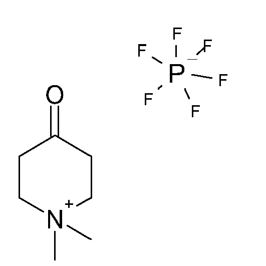

6,7 g N,N-Dimethylpiperidonbromid werden in 30 ml Wasser gelöst und durch Zugeben einer Lösung aus 5,9 g Natriumhexafluorophosphat in 30 ml Wasser zur Kristallisation gebracht. Die Kristalle werden filtriert, mit Wasser gewaschen und getrocknet. Ausbeute: 8,8g (57%); Fp.: 220-221°C

### Beispiel 5: N-Methylscopolaminium hexafluorophosphat

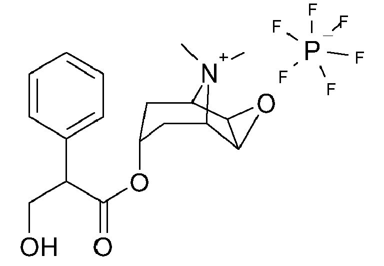

20 g N-Methylscopolaminium-Bromid werden in 200 ml Wasser gelöst und mit einer Lösung von 9,2 g Natriumhexafluorophosphat in 50 ml Wasser versetzt (25°C). Die ausgefallenen Kristalle werden mit 50 ml Wasser gewaschen und getrocknet. Ausbeute: 23,3 g (83%)

### Beispiel 6: Cholin hexafluorophosphat

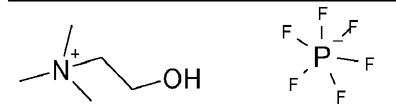

14 g Cholinchlorid werden in 50 ml Wasser gelöst und durch Zugeben einer Lösung aus 16,8 g Natriumhexafluorophosphat 30 ml Wasser zur Kristallisation gebracht. Die Kristalle werden filtriert, mit Wasser gewaschen und getrocknet. Ausbeute: 24,9g (60%); Fp.: 257°C

Die nachfolgenden Synthesebeispiele zeigen, dass die Verwendung von Hexafluorophosphaten der Formel **1** die einfache Durchführung von Synthesen mit Ammoniumverbindungen erlaubt.

### Beispiel 7: N-Methyl-endo-epoxytropanolium hexafluorophosphat

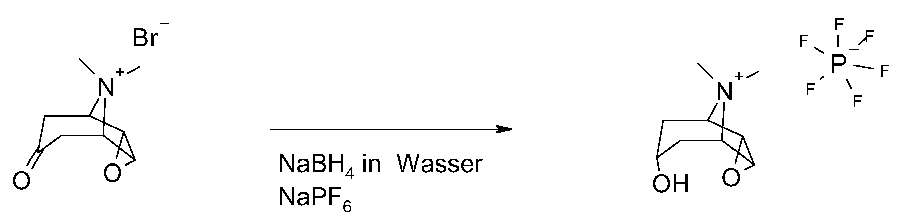

3,3 g N-Methyl-endo-epoxytropanonium Bromid wurden in 33 ml Wasser gelöst und innerhalb von 1 Stunde unter Kühlung mit 1 g NaBH4 und etwas HCOOH versetzt. Nach vollständiger Umsetzung wurden 2,5 g NaPF₆ zugegeben. Die ausgefallenen Kristalle werden abgesaugt, gewaschen und getrocknet. Ausbeute: 4,2g (43%)

Da das Hexafluorophosphat durch Kristallisation dem Gleichgewicht entzogen wird, gelingt diese Reaktion in einfacher Weise.

Das nachfolgende Beispiel dient der Illustration, wie in Wasser schwerlösliche Hexafluorophosphate der Formel **1** in einfacher Art und Weise in wasserlösliche Salze überführt werden können.

### Beispiel 8: Rückführung in das wasserlösliche Salz

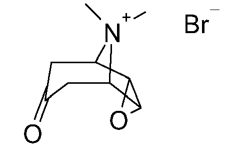

5,5g N-Methyl-endo-epoxytropanonium-hexafluorophosphat warden in 50 ml Aceton gelöst und mit einer Lösung von 1,8g LiBr in 20 ml Aceton versetzt. Die ausgefallenen Kristalle werden abgesaugt, gewaschen und getrocknet. Ausbeute 4,4g (85%); Fp. 200-202 °C;

Die nachfolgenden Beispiele illustrieren am Beispiel des Tiotropiumbromids, wie komplexe pharmazeutische Wirkstoffe mit Hilfe der erfindungsgemäßen Hexafluorophosphate schonend und unter vereinfachten Bedingungen erhalten werden können.

### Beispiel 9: N-Methylscopinium hexafluorophosphat

N-Methylscopiniumbromid wird in Wasser gelöst und mit einer equimolaren oder molar überschüssigen Menge eines in Wasser löslichen Hexafluorophosphates (Natrium- oder Kaliumsalz) versetzt. Auch wässrige Hexafluorophosphorsäure führt zur Ausfällung. Das N-Methylscopinium-hexafluorophosphat fällt / kristallisiert als weißes, wasserunlösliches Produkt aus, wird isoliert, gegebenenfalls mit Methanol gewaschen und dann bei ca. 40°C im Trockenschrank getrocknet.
Schmp.: 265-267°C (Schmelze unter Verfärbung);
H-NMR: in Acetonitril-d3 σ(ppm): 1,9 (dd, 2H) , 2,55( dd, 2H), 2,9 (s,3H), 3,29 (s,3H), 3,95(dd, 4H), 3,85 (s, 1H).

### Beispiel 10: Tiotropiumbromid

1,6 g (5mmol) Methylscopinium Hexafluorophoshat (Beispiel 10) und 2,0 g (7,8 mmol) Dithienylglycolsäuremethylester werden in 50 ml Aceton und in Gegenwart von 10g Molsieb 4A 50-70 Stunden unter Rückfluß gekocht.
Das Reaktionsgemisch wird filtriert, das Filtrat mit einer Lösung aus 0,3 g LiBr in 10 ml Aceton versetzt. Das auskristallisierte noch nicht umgesetzte N-Methylscopiniumbromid wird durch Filtration abgetrennt. Nach Zugabe von weiteren 0,6 g LiBr (gelöst in Aceton) fällt Tiotropiumbromid in einer isolierten Ausbeute von 30% (bezogen auf eingesetzte Verbindung nach Beispiel 9) aus.

### Beispiel 11: Tiotropiumhexafluorophosphat

Tiotropiumhexafluorophosphat wird im Rahmen der Umsetzung nach Beispiel 10 nicht isoliert sondern direkt weiter zum Tiotropiumbromid umgesetzt.
Für die Zwecke der Charakterisierung des Tiotropiumhexafluorophosphat wurde dieses spezifisch hergestellt und isoliert. Hierbei wurden die folgenden charakterisierenden Daten erhalten. Schmp.: 233-236°C (Schmelzen unter Verfärbung)
H-NMR: in aceton-d6 : σ(ppm): 2,08 (dd, 2H) , 2,23( dd, 2H), 3,32 (s,3H), 3,50 (s,3H), 3,62(s,2H), 4,28(m, 2H), 5,39(m, 1H) ,6,25 (s), 7,02(m,2H), 7,027,22(m,2H), 7,46(m,2H), P-NMR: in aceton-d6 : σ(ppm): -143,04, Heptett, J =4,37.

### Beispiel 12: Tiotropiumbromid

31,5 g (100mmol) Methylscopinium Hexafluorophoshat (Beispiel 9) und 25,4 g (100 mmol) Dithienylglycolsäuremethylester werden in 400 ml Aceton und in Gegenwart von 40g Molsieb 4A, Pulver (Fluka) und DMAP (4,4-Dimethylaminopyridin) 24h unter Rückfluß gekocht. (Molsieb wurde nach 3h in gleicher Menge ausgetauscht.)
Das Reaktionsgemisch wird filtriert, mit 200ml Aceton nachgewaschen, das Filtrat schrittweise mit einer Lösung aus 9,6 g LiBr (110mmol) in 110 ml Aceton versetzt. Das auskristallisierte noch nicht umgesetzte N-Methylscopiniumbromid wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Tiotropiumbromid in einer isolierten Ausbeute von 16,6g (35%) (bezogen auf eingesetzte Verbindung nach Beispiel 9) aus. Reinheit HPLC> 99%. Reinheit DC: keine Verunreinigungen erkennbar.

### Beispiel 13: Tiotropiumbromid

1,6 g (5 mmol) Methylscopinium Hexafluorophoshat (Beispiel 9) und 1,25 g (5 mmol) Dithienylglycolsäuremethylester werden in 50 ml Aceton und in Gegenwart von 2g Molsieb 4A, Pulver (Fluka) und 6mg Kalium-tert.-butylat bei 0°C 4 h lang gerührt.
Das Reaktionsgemisch wird filtriert, mit 20ml Aceton nachgewaschen, das Filtrat schrittweise mit einer Lösung aus 0,7 g LiBr (13mmol) in 11 ml Aceton versetzt. Das auskristallisierte noch nicht umgesetzte wird durch Filtration abgetrennt. (Fraktionierte Fällung). Die Kristallfraktionen wurden abfiltriert und getrocknet. Die Zusammensetzung der Fraktionen wurde dünnschichtchromatographisch bestimmt. Die Tiotropiumbromid-Fraktionen wurden abgesaugt, mit Aceton nachgewaschen, aus Wasser umkristallisiert, mit Aceton nachgewaschen und getrocknet. 1,2g (48% Ausbeute bezogen auf eingesetzte Verbindung nach Beispiel 9) Tiotropiumbromid wurden auf diese Weise isoliert.
Reinheit HPLC: 99,8% , DC: keine Verunreinigung sichtbar;

### Beispiel 14: Tiotropiumbromid

31,5g (0,1 mol) Methylscopinium Hexafluorophoshat (Beispiel 9) und 30,5g (0,10mol) 2,2'-Dithienylglycolsäuremethylester werden in 400 ml Aceton gelöst und in Gegenwart von 90g Zeolith vom Typ 4A (Na₁₂Al₁₂Si₁₂O₄₈ x n H₂O) und 0,2g (1mmol) Kalium-tert.-butylat über einen Zeitraum von 20-24 Stunden bei 0°C gerührt.
Das Reaktionsgemisch wird filtriert, das Filtrat mit einer Lösung aus 8,7 g LiBr (8,7 g 0,10mol in 100 ml Aceton) versetzt.
Das dabei auskristallisierte Produkt wird durch Filtration abgetrennt, mit Aceton gewaschen und dann getrocknet.
Man erhält 41,4 g (87,7%) Ausbeute, bei 90% Umsetzungsgrad.

Die beispielhaft aufgeführten Reaktionen erfolgen praktisch ohne Bildung von Nebenprodukten.. Sollten die Reaktionen gegebenenfalls ohne vollständige Umsetzung der Ausgangsmaterialien erfolgen, kann das im ersten Schritt der Aufarbeitung isolierte N-Methylscopiniumbromid daher mit der Reaktion nach Beispiel 10 zurückgeführt und damit die Gesamtausbeute im Rahmen eines Herstellverfahrens signifikant erhöht werden.

## Patentansprüche

1. Verfahren zur Herstellung von Ammoniumhexafluorophosphaten der Formel **1** 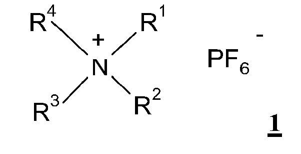 worin
R¹ und R² gleich oder verschieden Wasserstoff oder ein Rest ausgewählt aus der Gruppe bestehend C₁-C₁₀-Alkyl, C₂-C₁₀-Alkenyl, C₂-C₁₀-Alkinyl, C₃-C₈-Cycloalkyl, C₄-C₈-Cycloalkenyl, C₆-C₈-Cycloalkinyl, C₆-C₁₀-Aryl-C₁-C₆-alkyl, C₆-C₁₀-Aryl-C₂-C₆-alkenyl, C₆-C₁₀-Aryl-C₂-C₆-alkinyl, C₆-C₁₀-Aryl und Heterocyclyl, der gegebenenfalls substituiert sein kann;
R³ und R⁴ gleich oder verschieden Wasserstoff oder ein Rest ausgewählt aus der Gruppe bestehend C₁-C₁₀-Alkyl, C₂-C₁₀-Alkenyl, C₂-C₁₀-Alkinyl, C₃-C₈-Cycloalkyl, C₄-C₈-Cycloalkenyl, C₆-C₈-Cycloalkinyl, C₆-C₁₀-Aryl-C₁-C₆-alkyl, C₆-C₁₀-Aryl-C₂-C₆-alkenyl, C₆-C₁₀-Aryl-C₂-C₆-alkinyl, C₆-C₁₀-Aryl und Heteroaryl, der gegebenenfalls substituiert sein kann, oder
R³ und R⁴ gemeinsam mit dem Stickstoff ein mono-, bi- oder tricyclischer, gesättigter oder ungesättigter Carbocyclus der 4 bis 10 Kohlenstoffzentren enthalten kann, wobei gegebenenfalls ein oder zwei dieser Kohlenstoffzentren durch O oder S ersetzt sein können, und der gegebenenfalls substituiert sein kann;
**dadurch gekennzeichnet, dass** eine Verbindung der Formel 2 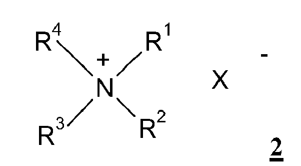 in der R', R², R³ und R⁴ die vorstehend für Verbindung **1** genannten Bedeutungen haben und worin
X⁻ ein einfach negativ geladenes Anion bedeuten kann,
in einem geeigneten Lösemittel durch Umsetzung mit einem Salz Kat⁺PF⁶⁻, wobei Kat⁺ für ein Kation ausgewählt aus der Gruppe bestehend aus Li⁺, Na⁺, K⁺, Mg2+, Ca2+, steht, in die Verbindung der Formel **1** überführt wird,
mit der Maßgabe, dass die Verbindung der Formel **1** nicht die Verbindung der Formel **1'** 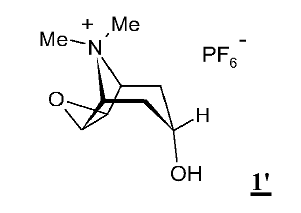 sein kann.

2. Verfahren nach Anspruch 1, **dadurch gekennzeichnet, dass** mit einem Salz Kat⁺PF₆⁻ erfolgt, in dem Kat⁺ ausgewählt ist aus der Gruppe bestehend aus Li⁺, Na⁺ und K⁺, besonders bevorzugt Na⁺ und K⁺.

3. Verfahren nach Anspruch 1 oder 2, **dadurch gekennzeichnet, dass** die Umsetzung in einem polare Lösemittel, bevorzugt in einem Lösemittel ausgewählt aus der Gruppe bestehend aus Wasser, Methanol, Ethanol, Propanol und Isopropanol, bevorzugt Wasser oder Methanol oder Mischungen davon, durchgeführt wird.

4. Ammonium-hexafluorophosphate der allgemeinen Formel **1**, 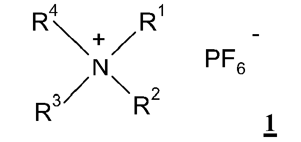 worin R¹, R², R³ und R⁴ die in Anspruch 1 genannten Bedeutungen haben können, mit der Maßgabe, dass es sich bei der Verbindung der Formel **1** nicht um Tiotropiumhexafluorophosphat oder um die Verbindung der Formel **1'** 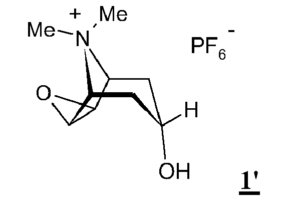 handeln kann.
